Europäisches Patentamt

European Patent Office

Office européen des brevets

(19)

(11) Publication number: 0 163 266
A2

(12) **EUROPEAN PATENT APPLICATION**

(21) Application number: 85106359.4

(22) Date of filing: 24.05.85

(51) Int. Cl.⁴: **G 02 B 23/26**

(30) Priority: 26.05.84 JP 107267/84
09.11.84 JP 236070/84

(43) Date of publication of application:
04.12.85 Bulletin 85/49

(84) Designated Contracting States:
DE FR GB

(71) Applicant: Hashimoto, Daijo
2-8-7, Nishikata Bunkyo-ku
Tokyo(JP)

(71) Applicant: HITACHI CABLE, LTD.
1-2, Marunouchi 2-chome
Chiyoda-ku Tokyo 100(JP)

(71) Applicant: Fuji Photo Optical Co., Ltd.
1-324 Uetake-cho Omiya-shi
Saitama-ken(JP)

(72) Inventor: Hashimoto, Daijo
2-8-7 Nishikata, Bunkyo-ku
Tokyo(JP)

(72) Inventor: Suzuki, Toshio
2-1-2 Marunouchi, Chiyoda-ku
Tokyo(JP)

(72) Inventor: Abe, Koichi
5-1 Hidaka-cho, Hitachi-shi
Ibaraki(JP)

(72) Inventor: Suzuki, Masane
1-324, Uetake-cho, Omiya-shi
Saitama(JP)

(72) Inventor: Kanaya, Motonori
1-324, Uetake-cho, Omiya-shi
Saitama(JP)

(72) Inventor: Saito, Syuki
2-1-2 Marunouchi, Chiyoda-ku
Tokyo(JP)

(72) Inventor: Shibamoto, Hiroshi
1-324, Uetake-cho, Omiya-shi
Saitama(JP)

(74) Representative: Hering, Hartmut, Dipl.-Ing.
Patentanwälte Dr. Thomson - Weinkauff - Hering P.O.
Box 701929
D-8000 München 70(DE)

(54) Optical fiber for lateral irradiation of laser beam.

(57) An optical fiber (10, 20, 30) for lateral irradiation of laser beam for making the laser beam to irradiate laterally in a through-endoscopic manner in a tubular cavity of an internal organ.

According to the present invention, the fiber (10, 20, 30) for the laser beam lateral irradiation has an exiting end (13, 23, 33) thereof formed into a reflectory surface (22, 32) inclined at about 45° to the center axis of the fiber (20, 30), and an exiting planar surface (23, 33) being in parallel to the center axis of the fiber (20, 30) is formed at a surface of the fiber (20, 30) on the exiting side of the laser beam reflected by the inclined reflectory surface (22, 32). With the laser beam lateral irradiation fiber (20, 30) according to the present invention, the cross-sectional shape of the exiting beam is circular and the scattering of the exiting beam is controlled, so that the irradiation beam high in density of the irradiation energy can be obtained. Furthermore, according to the present invention, a coil-shaped spring (60) is wound around the outer periphery of the fiber (10, 20, 30), so that the fiber (10, 20, 30) can be smoothly inserted through a very small hole such as a forceps hole (54) of a manual control section (40) of an endoscope.

./...

EP 0 163 266 A2

# FIG.3

## (A)

## (B)

# FIG.9

Dr. Thomsen · Weinkauff · Hering
D-8000 München 70, P.O. Box 701929

0163266

Daijo Hashimoto, Bunkyo-ku, Tokyo, Japan,
Hitachi Cable, Ltd., Chiyoda-ku, Tokyo, Japan und
Fuji Photo Optical Co., Ltd., Omiya-shi, Saitama, Japan

OPTICAL FIBER FOR LATERAL IRRADIATION OF LASER BEAM

BACKGROUND OF THE INVENTION

1. Field of the Invention

This invention relates to a fiber for the irradiation by a laser beam, and more particularly to the fiber for the lateral irradiation by the laser beam in a through-endoscopic manner in a tubular cavity of an internal organ.

2. Description of the Prior Art

A high speed development in the laser techniques have realized the treatment of an affected portion such as a tumor in a tubular cavity of an internal organ by the irradiation of the laser beam in a through-endoscopic manner. The fiber for introducing the laser beam into the tubular cavity of an internal organ of a living body to allow the laser beam to irradiate an affected portion in a through-endoscopic manner is a so-called direct irradiation fiber having an exiting end face perpendicularly intersecting the lengthwise direction thereof. When the direct irradiation fiber is used in a through-endoscopic manner, the endoscope is limited in its movement or fixed in the narrow tubular cavity of the internal organ, whereby such disadvantages are presented that, even though an affected condition is observed within the visual field of observation through the endoscope, the affected portion cannot be irradiated by the laser beam or a perfect irradiation of the affected portion cannot be carried out due to a deformation of the affected portion and therearound.

Furthermore, when the direct irradiation <span>0163266</span>
used together with a direct-view endoscope, the laser beam is
irradiated in a direction tangential to the affected portion
, whereby such ununiformities in the irradiation cannot be
avoided that a close-in portion is irradiated by an excessively
strong energy and a far-away portion is irradiated by an
insufficient energy, thus obtaining an ununiform distribution
of the irradiation energy to the affected portion. The ununiformity
in distribution of the irradiation energy presents a serious
problem in the treatment of a tumor and the like by the laser
beam from the clinical viewpoint.

The uniformity in distribution of the irradiation
energy is realized by the irradiation of the front of the
affected portion. To achieve this, there have been made attempts
to irradiate the front of the affected portion by use of so-
called obligue-view endoscope and side-view endoscope. However
, these obligue-view and side-view endoscopes are considerably
narrow in the visual field of inspection as compared with the
direct-view endoscope, and consequently, these obligue-view
and side-view endoscopes should not necessarily be effective
means as viewed from the easiness in search and inspection of
the affected portion, and further, the easiness in treatment
by the additional use of the fiber for irradiation by the laser
beam.

Further, the laser beam transmission fiber for the
treatment is very small in diameter and comparatively long.
It is most difficult that the optical fiber in the above-

described form is inserted through a forceps hole as being a very small diameter hole in an endoscope insertion section and made to successfully reach the forward end of the endoscope insertion section. Particularly, the forward end of the endoscope insertion section can be freely curved such that the forward end can be directed toward the affected portion. Thus , the curved form of the forward end of the endoscope insertion section makes it difficult to insert the optical fiber, More specifically, there are such possibilities that, when the optical fiber for the laser treatment is inserted through the forceps hole, the optical fiber cannot be smoothly inserted due to the frictional force generated between the outer peripheral surface of the optical fiber and the forceps hole , or, when the optical fiber is excessively forcedly inserted through the forceps hole, the optical fiber may be broken in the forceps hole.

## SUMMARY OF THE INVENTION

The present invention has as its object the provision of fiber for the lateral irradiation of a laser beam easily used for inspection and capable of irradiating the front of an affected portion in a tubular cavity of an internal organ by use of the laser beam.

According to the present invention, a fiber for the laser beam irradiation have an exiting end thereof formed in to a reflectory surface inclind at about 45 ° to the center axis of the fiber, whereby the beam path of the laser beam

3

0163266

transmitted through the fiber is reflected and curved so as to exit laterally from the side surface of the fiber. Further , the surface of the fiber on the exiting side is formed into an exiting planar surface in parallel to the center axis of the fiber, whereby the contour of the cross-section of the exiting beam comes to be circular and the scattering of the exiting beam is controlled, so that the irradiation beam high in density of the irradiation energy can be obtained.

Furthermore, another object of the present invention is to provide the fiber for transmitting the laser beam, capable of being smoothly inserted through a very small diameter hole such as a forceps hole in a manual control section of an endoscope.

To achieve the above-described objects, the present invention contemplates that a coil-shaped spring is wound around the outer periphery of the optical fiber constituted by a core and a clad, which are different in refractive index from each other.

## BRIEF DESCRIPTION OF THE INVENTION

The exact nature of this invention, as well as other objects and advantages thereof, will be readily understood from consideration of the following specification relating to the accompanying drawings, like reference characters designate the same or similar parts throughout the figures thereof and wherein:

Figs. 1A and 1B are a sectional view and a front

0163266

view showing the fiber for transmitting the beam, respective-
ly;

Figs. 2A and 2B are a sectional view and a front
view showing an example of the optical fiber for the lateral
irradiation, respectively;

Figs. 3A and 3B are a sectional view and a front
view showing an embodiment of the optical fiber for the lateral
irradiation according to the present invention, respectively
;

Figs. 4 to 6 are graphic charts of the measured
values showing the distance-beam size of the exiting beam of
the optical fiber shown in Figs. 1 to 3, respectively;

Fig. 7 is a schematic diagram showing the general
configuration of the endoscope;

Fig. 8 is an enlarged front view showing the manual
control section of the endoscope;

Fig. 9 is a schematic diagram showing the fiber for
transmitting the laser beam of this embodiment;

Fig. 10 is an enlarged sectional view showing the
fiber of this embodiment shown in Fig. 9; and

Fig 11 is an explanatory view showing the use condition
of this embodiment.

DETAILED DESCRIPTION OF THE INVENTION

Description will hereunder be given of the preferred
embodiment of the fiber for the lateral irradiation by the
laser beam according to the present invention with reference to the accompanying drawings.

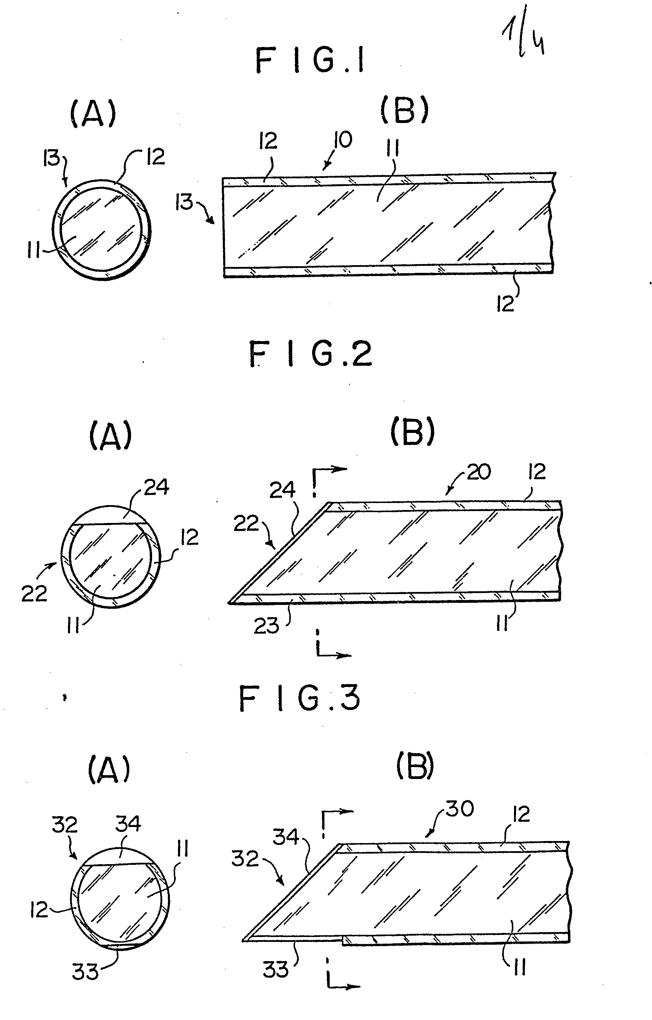

To facilitate the understanding of the present invention , conventional direct irradiation fiber and one example of the lateral irradiation fiber will now be described with reference to Figs. 1 and 2.

Figs 1A and 1B are the sectional view and the front view showing one portion of the exiting end of the fiber 10 for transmitting a beam, constituted by a core 11 and a clad 12, which are different in refractive index from each other, and the fiber is a well known one. This fiber is made of silica (quartz), has a core diameter of $600\mu$, the outer diameter of a clad layer of $750\ \mu$, a difference in refractive index of about 1.5 % and the number of openings at the falling-in end $NA = 0.25$. The above-described fiber is referred to a so-called direct irradiation fiber.

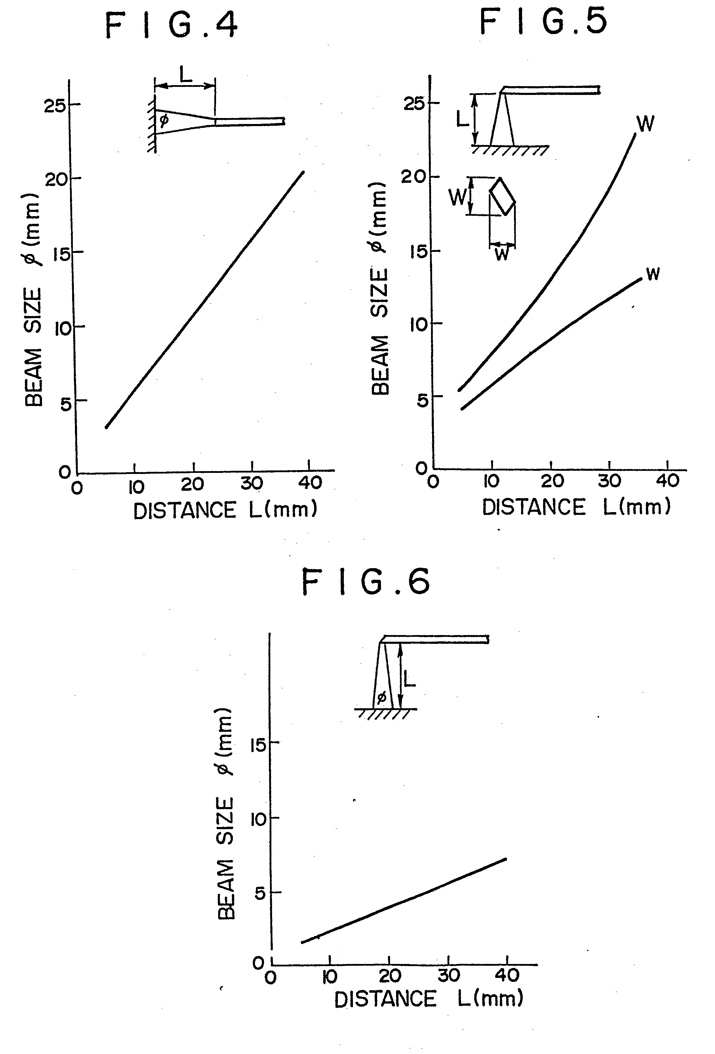

When the beam flux such for example as He-Ne laser beam flux, which has fallen into the fiber 10, exits from an exiting end 13 of the fiber 10, the beam flux is scattered in a manner to be increased in its diameter $\phi$ substantially in direct proportion to a distance L from the exiting end 13 as shown in Fig. 4, namely, in conical shape. Fig. 4 shows the measured value, which substantially meets the theoretical value.

Figs. 2A and 2B show the construction of the exiting end of the fiber, which is thought of for curving the beam flux exiting from the fiber laterally relative to the lengthwise direction of the fiber. In Fig.2, the end race of the fiber 20 is formed into a planar surface 22 inclined at about 45 °

to the center axis of the fiber 20. Further, the inclined planer surface 22 is deposited thereon with a reflectory film layer 24 formed of a reflectory film material such as silver or aluminum by a known vacuum deposition method for example. This is quite similar to the fiber shown in Fig. 1, except that a reflectory surface is formed such that the optical axis of the transmitting beam path of the fiber 20 is bent laterally at a right angle

. The beam flux, which has fallen into the fiber 20 of the above-described construction, is reflected at the inclined reflectory planar surface 22, and thereupon, exits laterally , making a portion of the side surface of the cylindrical fiber as an exiting end 23. However, the exiting end 23 of the above-described lateral irradiation fiber 20 has an optical function similar to a cylindrical lens, whereby the exiting beam flux makes different scatterings in two directions perpendicularly intersecting each other. As the result of measuring the exiting beam flux under the same conditions as the case of the fiber 10 shown in Fig. 1, it is understood that the difference in degree of the scatterings in the two directions W-w in considerably increased with the increase in the distance L as shown in Fig. 5.

With the fiber 20 having the construction of the exiting end as shown in Fig. 2, the lateral irradiation, namely , the irradiation of the front of the affected portion can be realized, however, the distribution of the irradiaion energy becomes ununiform depending upon the directions. Particularly , at a position where it is difficult to bring the exiting end of the laser beam irradiation fiber being present in the tubular cavity toward the affected portion, ununiformity in distrubution of the energy is considerably increased. It is understood that the above fact is not preferable.

Now, Figs. 3A and 3B are the sectional views showing a preferable embodiment of the fiber for the lateral irradiation by the laser beam according to the present invention. In Fig.3 , a planar surface 32 inclined at about 45° to the center axis is of the fiber 30 is formed at the end of the fiber 30, which planar surface is further vacuum deposited thereon with a reflectory film layer 34. This is quite similar to the fiber 20 shown in Fig. 2. However, an exiting planar surface 33 being in parallel to the center axis of the fiber 30 is formed at an exiting portion of the beam flux reflected by the inclined reflectory planar surface 32. It is desirable that this exiting planar surface 33 should be formed such that a clad layer of $15 \sim 20\mu$ remains between the core 11 of the fiber 30 and itself. When the clad layer remains between the exiting planar surface 33 and the core, such a disadvantage can be avoided that part of the beam reaching the exiting planar surface 33 from the beam transmitted through the fiber penetrates forwardly in the longitudinal direction of the fiber 30 before the aforesaid beam reach the reflectory planar surface 32 to thereby lower the efficiency of transmission of the fiber.

The laser beam exiting from the fiber 30 for the lateral irradiation, which forms the aforesaid exiting planar surface 33, becomes circular in the cross-sectional shape, is decreased in the degree of scattering and lowered in the directional properties of the scattering. As the result, the ununiformity in the irradiation energy due to the positions of the affected portion disappears. Fig. 6 is the graphic chart of the measured values showing the relationship between a distance L and a beam size $\phi$ of the exiting beam of the fiber30 for the lateral irradiation under the same conditions as in the cases of Figs. 4 and 5, in which the directional properties of the scattering as observed in the lateral irradiation fiber 20 of Fig. 2 are satisfactorily improved, and the degree of scattering is decreased as compared with the direct irradiation fiber 10 in Fig. 1.

In the above embodiment, the inclined reflectory surface has been formed into the planar surface, but can be formed into a spherical surface as necessary.

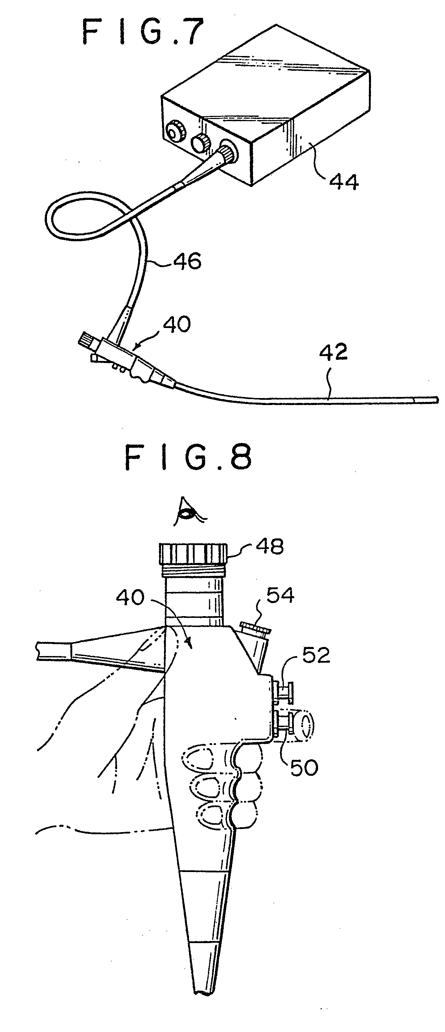

Figs. 7 to 11 disclose the techniques of smoothly inserting the above-described fiber for transmitting the laser beam through a very small diameter hole such as a forceps hole in the manual control section of the endoscope.

Fig. 7 shows a commonly used endoscope including a manual control section 40, a flexible insertion section 42 connected to this manual control section 40, for being inserted into a deep portion in a living body or the like, and a connecting section 46 for connecting the manual control section 40 to a control unit 44 incorporating therein a light source neccessary for this endoscope and other various control mechanisms. As enlargedly shown in Fig. 8, the manual control section 40 is provided on the top thereof with an eye piece portion 48 including an eyepiece, at the front thereof with a air-water feed control button 50, a suction control button 52 and a forceps insertion opening 54. The fiber for transmitting the laser beam, formed at the forward end thereof with a laser scalpel section is normally inserted through the forceps insertion opening 54, passed through the manual control section 40 and the insertion section 42 and exposed from the forward end of the insertion section 42.

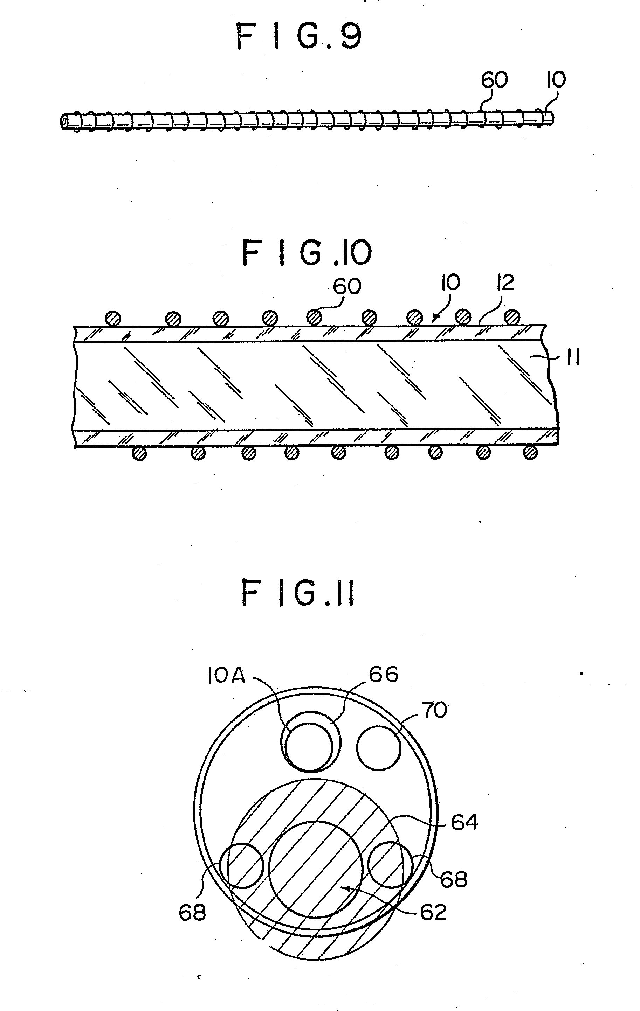

As shown in Fig. 9, a coil-shaped spring 60 is wound around the outer periphery of the fiber 10 according to the present invention. As aforesaid, the fiber 10 is constituted by the core 11 and the clad 12 disposed around the outer periphery of the core 11 and formed of a quartz clad or a polymer clad, and the core 11 and the clad 12 are different in refractive index from each other. Furthermore, the outer periphery of the clad 12 is applied with a silicone coating, and further, the outer periphery of the silicone coating may be protected by a covering layer made of nylon in some cases . These coating and covering layer are applied in accordance with the purposes for improving the reinforcement and mechanical strength of the optical fiber.

As the material of the coil-shaped spring 60, there are used a high tensile strength steel wire, a piano wire, a phosphor bronze spring material, a rigid copper wire, a stainless steel spring material or the like. Particularly, the stainless

0163266

steel spring material is useful because of its rust-proof function.

As the method of winding the coil-shaped spring 60 around the outer periphery of the optical fiber 10, there are following methods. Firstly, a spring is wound around the outer periphery of the optical fiber 10, to thereby obtain the coil-shaped spring 60 wound around the outer periphery of the optical fiber 10. A second method is that the optical fiber 10 is inserted through a hollow space in the spring 60, which has previously been wound into a coil-shape, to thereby obtain the coil-shaped spring 60 wound around the outer periphery of the opticcal fiber 10. A third method is that the optical fiber 10 is inserted through a hollow space in the spring 60 being shorter than the optical fiber 10, which has been previously wound into a coil-shape, thereafter, the spring 60 is forcedly extended, and opposite ends thereof are solidly secured to the fiber 10 by staking and the like, to thereby obtain the coil-shaped spring 60 wound around the outer periphery of the optical fiber 10. This method is advantageous in that the spring 60 is closely attached to the outer periphery of the optical fiber 10. The number of the coil-shaped springs 60 wound around the outer periphery of the optical fiber may be one or a plurality of springs. Furthermore, the spring 60 may be either close-coiled or normally coiled. Additionally, the cross-sectional shape of the coil-shaped spring 60 need not necessarily be limited to a circular one, but may be an elliptical or rectangular shape.

Connection between the fiber 10 and the spring 60 may be made such that the opposite ends of the spring 60 may be fixed by staking, or the both members may be connected to each other by fusion bonding or the like through the heat of an external material of the optical fiber such as glass or synthetic resin. The connection therebetween may be made by use of a bonding agent.

The following is action of the embodiment of the laser beam transmitting fiber according to the present invention with the above-described arrangement. Firstly, as shown in Fig. 9, the fiber 10, the outer periphery of which is wound around by the coil-shaped spring 60, is inserted through the forceps hole 54 of the manual control section 40 of the endoscope as shown in Fig. 8. In this case, with the conventional fiber , the forceps hole have been in line-to-line contact with the outer peripheral surface of the fiber, however, with the fiber 10 according to this embodiment, the fiber 10 is in point-to-point contact with the forceps hole because the coil-shaped spring 60 is wound around the fiber 10. Because of this, the frictional force imposed on the fiber 10 is decreased when inserted, and, even into the insertion section 42 with a curved forward end, the fiber can be smoothly inserted. Furthermore , when the laser scalpel at the forward end of the fiber is required to be replaced with new one due to the damage and the like thereof, the replacement may be quickly carried out because the fiber can be easily drawn out. Further, the outer periphery of the fiber 10 is protected by the spring 60, so that breakages of the laser scalpel may occur fewer than in the case where the laser scalpel is used only with the fiber .

Further, the outer periphery of the fiber 10 is wound around by the spring 60, whereby the follow-up properties of the fiber 10 are improved, so that the laser scalpel portion at the forward end of the fiber 10 can be readily directed to a desired position. More specifically, as shown in Fig. 11, when the visual field of an objective lens 62 at the forward end of the insertion section of the endoscope is included within a scope indicated by hatching 64, it is desired to direct a laser scalpel section 10A exposed from a forceps hole 66 within the hatched scope 64. In such case, the follow-up properties of the conventional fiber have been unsatisfactory. However, the fiber according to the present invention, interposing the spring 60 between the fiber and the forceps hole, has higher follow-up properties, so that the laser scalpel can be readily directed with in the scope of visual field of the objective lens. Additionally, in Fig. 11, designated at 68 is a light guide, and 70 an air-water feed channel.

As has been described hereinabove, with the fiber for the lateral irradiation of laser beam according to the present invention, the portions, which have been unable to irradiate by the conventional direct irradiation fiber, can be irradiated, and an affected portion in a narrow tubular cavity can be uniformly iradiated from the front thereof. The fiber according to the present invention is high in durability

0163266

since such a possibility is eliminated that a damage of an optical element, particularly, due to an adhesion of a liquefied foreign material, which would occur when a refracted optical element of a very small size such for example as a microprism is attached to the forward end of the direct irradiation fiber . Further, these is such an advantage that, even though the fiber according to the present invention is used together with the direct-view endoscope, which is advantageous in the observation performance in the tubular cavity, the lateral irradiation fiber is moved back and forth or rotated with the large visual field being held unmoved, so that the portion to be irradiated can be easily selected.

According to the present invention, the coil-shaped spring is wound around the outer periphery of the optical fiber constituted by the core and the clad, which are different in refractive index from each other, so that the fiber can be easily inserted through the forceps hole and the like of the endoscope, thus offering such a disadvantage that insertion, removal, replacement and the like of the laser scalpel can be readily made.

Claims:

1. An optical fiber (20, 30) for lateral irradiation of laser beam wherein a reflectory surface (22, 32) inclined at about 45° to the center axis of a fiber (20, 30) is formed at an exiting end (23, 33) of the beam transmitting fiber (20, 30) constituted by a core (11) and a clad (12), which are different in refractive index from each other, and an exiting planar surface (23, 33) being parallel to the center axis of said fiber (20, 30) is formed at a surface of said fiber (20, 30) on the exiting side of the laser beam reflected by said inclined reflectory surface (22, 32).

2. An optical fiber (20, 30) for lateral irradiation of laser beam as set forth in claim 1, wherein a clad layer (12) remains between said exiting surface (23, 33) being in parallel to the center axis of said fiber (20, 30) and said core (11).

3. An optical fiber (20, 30) for lateral irradiation of laser beam as set forth in claim 1, wherein a reflectory film layer (24, 34) is provided on said inclined reflectory surface (22, 32).

4. An optical fiber (20,30) for lateral irradiation of laser beam, wherein a reflectory surface (22,32) inclined at about 45° to the center axis of a fiber (20,30) is formed at an exiting end (23,33) of the beam transmitting fiber (20,30) constituted by a core (11) and a clad (12), which are different in refractive index from each other, an exiting planar surface (23,33) being parallel to the center axis of said fiber (20,30) is formed at a surface of said fiber (20,30) on the exiting side of the laser beam reflected by said inclined reflectory surface (22,32) and a coil-shaped spring (60) is wound around the outer periphery of said fiber(20,30).

5.    An optical fiber (20, 30) for lateral irradiation of laser beam as set forth in claim 4, wherein said coil-shaped spring (60) is wound around the outer periphery of the optical fiber (20, 30).

6.    An optical fiber (20, 30) for lateral irradiation of laser beam as set forth in claim 4, wherein said spring (60) is previously formed into a coil-shape and the optical fiber (20, 30) is inserted through a hollow space in said coil-shaped spring (60) to obtain the coil-shaped spring (60) wound around the outer periphery of the optical fiber (20, 30).

7.    An optical fiber (20, 30) for lateral irradiation of laser beam as set forth in claim 6, wherein, after the optical fiber (20, 30) is inserted through the hollow space in said coil-shaped spring (60), said coil-shaped spring (60) is forcedly extended in the longitudinal direction to wind around the outer periphery of the optical fiber (20, 30).

8.    An optical fiber (20, 30) for lateral irradiation of laser beam as set forth in claim 4, wherein the outer peripheral surface of said optical fiber (20, 30) is connected to said coil-shaped spring (60) by fusion bonding through the heat of the external material of said optical fiber (20, 30).

9.    An optical fiber (20, 30) for lateral irradiation of laser beam as set forth in claim 4, wherein the outer peripheral surface of said optical fiber (20, 30) and said coil-shaped spring (60) are bonded to each other through a bonding agent.

10.  An optical fiber (20, 30) for lateral irradiation of laser beam as set forth in claim 4, wherein said clad (12) of the optical fiber (20, 30) is a quartz glass clad or a polymer clad.

FIG.1
(A)
(B)

FIG.2
(A)
(B)

FIG.3
(A)
(B)

0163266

1/4

FIG.4

FIG.5

FIG.6

0163266

# F I G.7

44

46

40

42

# F I G.8

48

40

54

52

50

# F I G . 9

60  10

# F I G .10

60   10  12

11

# F I G .11

10A   66   70

64

68   68

68   62